# EUROPEAN PATENT SPECIFICATION

(11) **EP 2 295 536 B1**
(45) Date of publication and mention of the grant of the patent: **06.01.2016**
(21) Application number: 09754672.5
(22) Date of filing: 26.05.2009
(51) Int. Cl.: C12N 1/21, C12N 15/09, C12P 7/62, C12N 9/04, C12N 9/10

(54) **MICROORGANISM CAPABLE OF PRODUCING IMPROVED POLYHYDROXYALKANOATE AND METHOD OF PRODUCING POLYHYDROXYALKANOATE BY USING THE SAME**
MIKROORGANISMUS MIT FÄHIGKEIT ZUR PRODUKTION VON VERBESSERTEM POLYHYDROXYALKANSÄUREESTER UND VERFAHREN ZUR HERSTELLUNG VON POLYHYDROXYALKANSÄUREESTER DAMIT
MICROORGANISME CAPABLE DE PRODUIRE DU POLYHYDROXYALCANOATE AMÉLIORÉ ET PROCÉDÉ DE PRODUCTION DE POLYHYDROXYALCANOATE À L'AIDE DE CELUI-CI

(30) Priority: 26.05.2008 JP 2008136356
(43) Date of publication of application: 16.03.2011
(73) Proprietor: Kaneka Corporation, Osaka (JP)
(72) Inventor: MURAKAMI, Hiroka, Takasago-shi Hyogo 676-8688 (JP); SATO, Shunsuke, Takasago-shi Hyogo 676-8688 (JP); FUJIKI, Tetsuya, Osaka-shi Osaka 530-8288 (JP)
(74) Representative: Vossius & Partner Patentanwälte Rechtsanwälte mbB
(86) International application number: PCT/JP2009/059564
(87) International publication number: WO 2009/145164

(56) References cited:
- WO-A1-2008/090873
- JP-A- H07 265 065
- JP-A- 2008 029 218
- JP-A- 2008 086 238
- JP-A- 2008 086 238
- SLATER STEVEN ET AL.: 'Multiple beta-Ketothiolases Mediate Poly(beta-Hydroxyalkanoate) Copolymer Synthesis in Ralstonia eutropha.' JOURNAL OF BACTERIOLOGY vol. 180, no. 8, 1998, pages 1979 - 1987, XP008138775
- FUKUI TOSHIAKI ET AL.: 'Engineering of Ralstonia eutropha for Production of Poly(3- hydroxybutyrate-co-3-hydroxyhexanoate) from Fructose and Solid-State Properties of the Copolymer.' BIOMACROMOLECULES vol. 3, 2002, pages 618 - 624, XP002538853
- FUKUI TOSHIAKI ET AL.: 'Cloning and Analysis of the Poly(3-Hydroxybutyrate-co-3- Hydroxyhexanoate) Biosynthesis Genes of Aeromonas caviae.' JOURNAL OF BACTERIOLOGY vol. 179, no. 15, 1997, pages 4821 - 4830, XP002113257
- FUKUI T. ET AL.: 'Efficient production of polyhydroxyalkanoates from plant oils by Alcaligenes eutrophus and its recombinant strain.' APPL. MIROBIOL. BIOTECHNOL. vol. 49, 1998, pages 333 - 336, XP002979648

### SEQUENCE LISTING

<110> Kaneka Corporation
<120>MICROORGANISM CAPABLE OF PRODUCING IMPROVED POLYHYDROXYALKANOATE AND METHOD OF PRODUCING POLYHYDROXYALKANOATE BY USING THE SAME
<130> B080188WO01-
<150> JP2008-136356
   <151> 2008-05-26
<160> 29
<170> PatentIn version 3.1
<210> 1
   <211> 481
   <212> DNA
   <213> Cupriavidus necator
<400> 1
   ccacgtagag ccagccaatg gccacgatgt acatcaaaaa ttcatccttc tcgcctatgc      60
   tctggggcct cggcagatgc gagcgctgca taccgtccgg taggtcggga agcgtgcagt     120
   gccgaggcgg attcccgcat tgacagcgcg tgcgttgcaa ggcaacaatg gactcaaatg     180
   tctcggaatc gctgacgatt cccaggtttc tccggcaagc atagcgcatg gcgtctccat     240
   gcgagaatgt cgcgcttgcc ggataaaagg ggagccgcta tcggaatgga cgcaagccac     300
   ggccgcagca ggtgcggtcg agggcttcca gccagttcca gggcagatgt gccggcagac     360
   cctcccgctt tgggggaggc gcaagccggg tccattcgga tagcatctcc ccatgcaaag     420
   tgccggccag ggcaatgccc ggagccggtt cgaatagtga cggcagagag acaatcaaat     480
   c                                                                     481
<210> 2
   <211> 385
   <212> DNA
   <213> Aeromonas caviae
<400> 2
   cgatctggac cggggtgctg gcctgggcca cgccggcgag ggccagcgcg gagcaaccga      60
   gcagcagggc gagaggtttc atcgggattc cttggcagtc tgaatgacgt gccagcctat     120
   cagcgcggcg ccggtgcggc gagggcgcgc cggacccagt gcgtcacctc tcgtctgatc     180
   cgcctccctc gacgggcgtc gctgacaaaa aaattcaaac agaaattaac atttatgtca     240
   tttacaccaa accgcatttg gttgcagaat gctcaaacgt gtgtttgaac agagcaagca     300
   acacgtaaac agggatgaca tgcagtaccc gtaagaaggg ccgattggcc cagatctcgc     360
   ctcgggtgtg ggtgaaggag agcac                                           385
<210> 3
   <211> 1362
   <212> DNA
   <213> Streptomyces cinnamonensis
<400> 3
   atgaaggaaa tcctggacgc gattcaggcc cagaccgcga ccgcgagcgg caccgccgcg      60
   gtcacgtccg ccgacttcgc cgctctcccc ctgcccgact cgtaccgcgc gatcaccgtg     120
   cacaaggacg agacggagat gttcgcgggc ctcgagtccc gtgacaagga cccccgcaag     180
   tcgctccatc tggacgacgt gccgatcccc gaactcggcc ccggtgaggc cttggtggcc     240
   gtcatggcct cctcggtcaa ctacaactcc gtgtggacct cgatcttcga gcccgtctcc     300
   accttcagct tcctggagcg gtacggccgg ctcagcgacc tgagcaagcg ccacgacctg     360
   ccgtaccaca tcatcggctc cgacctggcg ggcgtcgtgc tgcgcaccgg gcccggcgtg     420
   aacgcctgga acccgggcga cgaggtcgtc gcgcactgcc tgagcgtcga gctggagtcc     480
   tccgacggcc acaacgacac gatgctcgac cccgagcagc gcatctgggg cttcgagacc     540
   aacttcggcg gtctcgccga gatcgcgctc gtcaagtcca accagctcat gccgaagccc     600
   ggtcacctga gctgggagga ggccgcctcg cccggcctgg tgaactccac cgcgtaccgc     660
   cagctggtgt cccgcaacgg cgccggcatg aagcagggcg acaacgtgct gatctggggc     720
   gcgagcggcg gactcgggtc gtacgccacg cagttcgcgc tcgccggcgg cgccaacccc     780
   atctgtgtcg tctccagccc ccagaaggcg gagatctgcc gcgcgatggg cgccgaggcg     840
   atcatcgacc gcaacgccga gggctacaag ttctggaagg acgagcagac ccaggacccc     900
   aaggagtgga agcgcttcgg caagcgcatc cgcgagctca ccgggcggcg aggactcgac     960
   atcgtcttcg agcaccccgg ccgcgagacc ttcggcgcct cggtctacgt cacgcgcaag    1020
   ggcggcacca tcaccacctg cgcctcgacc tcgggctaca tgcacgagta cgacaaccgc    1080
   tacctgtgga tgtccctgaa gcgcatcatc ggctcgcact tcgccaacta ccgcgaggcg    1140
   tgggaggcca accgcctgat cgccaagggc aagatccacc cgacgctctc caagacgtac    1200
   cgcctggagg acaccggcca ggccgcctac gacgtccacc gcaacctcca ccagggcaag    1260
   gtcggcgtcc tcgccctcgc gcccgaggag ggcctgggcg tgcgcgaccc ggagaagcgg    1320
   gcccagcaca tcgacgcgat caaccgtttc cgcaacgtct ga                       1362
<210> 4
   <211> 1785
   <212> DNA
   <213> Artificial
<220>
   <223> Mutant of phaC derived from A.caviae
<400> 4
   atgagccaac catcttatgg cccgctgttc gaggccctgg cccactacaa tgacaagctg      60
   ctggccatgg ccaaggccca gacagagcgc accgcccagg cgctgctgca gaccaatctg     120
   gacgatctgg gccaggtgct ggagcagggc agccagcaac cctggcagct gatccaggcc     180
   cagatgaact ggtggcagga tcagctcaag ctgatgcagc acaccctgct caaaagcgca     240
   ggccagccga gcgagccggt gatcaccccg gagcgcagcg atcgccgctt caaggccgag     300
   gcctggagcg aacaacccat ctatgactac ctcaagcagt cctacctgct caccgccagg     360
   cacctgctgg cctcggtgga tgccctggag ggcgtccccc agaagagccg ggagcggctg     420
   cgtttcttca cccgccagta cgtcagcgcc atggccccca gcaacttcct ggccaccaac     480
   cccgagctgc tcaagctgac cctggagtcc ggcggccaga acctggtgcg cggactggcc     540
   ctcttggccg aggatctgga gcgcagcgcc gatcagctca acatccgcct gaccgacgaa     600
   tccgccttcg agctcgggcg ggatctggcc ctgaccccgg gccgggtggt gcagcgcacc     660
   gagctctatg agctcattca gtacagcccg actaccgaga cggtgggcaa gacacctgtg     720
   ctgatagtgc cgcccttcat caacaagtac tacatcatgg acatgcggcc ccagaactcc     780
   ctggtcgcct ggctggtcgc ccagggccag acggtattca tgatctcctg gcgcaacccg     840
   ggcgtggccc aggcccaaat cgatctcgac gactacgtgg tggatggcgt catcgccgcc     900
   ctggacggcg tggaggcggc caccggcgag cgggaggtgc acggcatcgg ctactgcatc     960
   ggcggcaccg ccctgtcgct cgccatgggc tggctggcgg cgcggcgcca gaagcagcgg    1020
   gtgcgcaccg ccaccctgtt cactaccctg ctggacttct cccagcccgg ggagcttggc    1080
   atcttcatcc acgagcccat catagcggcg ctcgaggcgc aaaatgaggc caagggcatc    1140
   atggacgggc gccagctggc ggtctccttc agcctgctgc gggagaacag cctctactgg    1200
   aactactaca tcgacagcta cctcaagggt cagagcccgg tggccttcga tctgctgcac    1260
   tggaacagcg acagcaccaa tgtggcgggc aagacccaca acagcctgct gcgccgtctc    1320
   tacctggaga accagctggt gaagggggag ctcaagatcc gcaacacccg catcgatctc    1380
   ggcaaggtga agacccctgt gctgctggtg tcggcggtgg acgatcacat cgccctctgg    1440
   cagggcacct ggcagggcat gaagctgttt ggcggggagc agcgcttcct cctggcggag    1500
   tccggccaca tcgccggcat catcaacccg ccggccgcca acaagtacgg cttctggcac    1560
   aacggggccg aggccgagag cccggagagc tggctggcag gggcgacgca ccagggcggc    1620
   tcctggtggc ccgagatgat gggctttatc cagaaccgtg acgaagggtc agagcccgtc    1680
   cccgcgcggg tcccggagga agggctggcc cccgcccccg gccactatgt caaggtgcgg    1740
   ctcaaccccg tgtttgcctg cccaacagag gaggacgccg catga                   1785
<210> 5
   <211> 1182
   <212> DNA
   <213> Cupriavidus necator
<400> 5
   atgactgacg ttgtcatcgt atccgccgcc cgcaccgcgg tcggcaagtt tggcggctcg      60
   ctggccaaga tcccggcacc ggaactgggt gccgtggtca tcaaggccgc gctggagcgc     120
   gccggcgtca agccggagca ggtgagcgaa gtcatcatgg gccaggtgct gaccgccggt     180
   tcgggccaga accccgcacg ccaggccgcg atcaaggccg gcctgccggc gatggtgccg     240
   gccatgacca tcaacaaggt gtgcggctcg ggcctgaagg ccgtgatgct ggccgccaac     300
   gcgatcatgg cgggcgacgc cgagatcgtg gtggccggcg gccaggaaaa catgagcgcc     360
   gccccgcacg tgctgccggg ctcgcgcgat ggtttccgca tgggcgatgc caagctggtc     420
   gacaccatga tcgtcgacgg cctgtgggac gtgtacaacc agtaccacat gggcatcacc     480
   gccgagaacg tggccaagga atacggcatc acacgcgagg cgcaggatga gttcgccgtc     540
   ggctcgcaga acaaggccga agccgcgcag aaggccggca agtttgacga agagatcgtc     600
   ccggtgctga tcccgcagcg caagggcgac ccggtggcct tcaagaccga cgagttcgtg     660
   cgccagggcg ccacgctgga cagcatgtcc ggcctcaagc ccgccttcga caaggccggc     720
   acggtgaccg cggccaacgc ctcgggcctg aacgacggcg ccgccgcggt ggtggtgatg     780
   tcggcggcca aggccaagga actgggcctg accccgctgg ccacgatcaa gagctatgcc     840
   aacgccggtg tcgatcccaa ggtgatgggc atgggcccgg tgccggcctc caagcgcgcc     900
   ctgtcgcgcg ccgagtggac cccgcaagac ctggacctga tggagatcaa cgaggccttt     960
   gccgcgcagg cgctggcggt gcaccagcag atgggctggg acacctccaa ggtcaatgtg    1020
   aacggcggcg ccatcgccat cggccacccg atcggcgcgt cgggctgccg tatcctggtg    1080
   acgctgctgc acgagatgaa gcgccgtgac gcgaagaagg gcctggcctc gctgtgcatc    1140
   ggcggcggca tgggcgtggc gctggcagtc gagcgcaaat aa                       1182
<210> 6
   <211> 31
   <212> DNA
   <213> Artificial
<220>
   <223> primer-1
<400> 6
   ggccgaattc cgatctggac cggggtgctg g 31
<210> 7
   <211> 20
   <212> DNA
   <213> Artificial
<220>
   <223> primer-2
<400> 7
   gtgctctcct tcacccacac 20
<210> 8
   <211> 33
   <212> DNA
   <213> Artificial
<220>
   <223> primer-3
<400> 8
   cggcggatcc gcgcatgaag cgcccggcca atg 33
<210> 9
   <211> 33
   <212> DNA
   <213> Artificial
<220>
   <223> primer-4
<400> 9
   ggccgaattc gactttgtct ccatgaggtt atg 33
<210> 10
   <211> 21
   <212> DNA
   <213> Artificial
<220>
   <223> primer-5
<400> 10
   atgacgcgtg aagtggtagt g 21
<210> 11
   <211> 30
   <212> DNA
   <213> Artificial
<220>
   <223> primer-6
<400> 11
   ggccatcgat ccttgaagta gccggccttg 30
<210> 12
   <211> 20
   <212> DNA
   <213> Artificial
<220>
   <223> primer-7
<400> 12
   ggtgatcgcc atcatcagcg 20
<210> 13
   <211> 20
   <212> DNA
   <213> Artificial
<220>
   <223> primer-8
<400> 13
   gatttgattg tctctctgcc 20
<210> 14
   <211> 23
   <212> DNA
   <213> Artificial
<220>
   <223> primer-9
<400> 14
   gactttgtct ccatgaggtt atg 23
<210> 15
   <211> 29
   <212> DNA
   <213> Artificial
<220>
   <223> primer-10
<400> 15
   ataacaattg cgatctggac cggggtgct 29
<210> 16
   <211> 122
   <212> DNA
   <213> Artificial
<220>
   <223> primer-11
<400> 16
   gaggatcgat gactttgtct ccatgaggtt atgttgcacc gcacagtata gcgggacgcg     60
   gacgaaatcg aacgagcgta cttttaccta atcaacccgt tgtgctctcc ttcacccaca    120
   cc                                                                   122
<210> 17
   <211> 35
   <212> DNA
   <213> Artificial
<220>
   <223> primer-12
<400> 17
   tattggatcc ccgtacccgc acgtcgacga gatca 35
<210> 18
   <211> 37
   <212> DNA
   <213> Artificial
<220>
   <223> primer-13
<400> 18
   attcaattgt cagaccgcgc cccacaggtc gtggcca 37
<210> 19
   <211> 95
   <212> DNA
   <213> Artificial
<220>
   <223> primer-14
<400> 19
   gtagatcgat actttgtccc cttgaggtta tgttgcaccg cacagtatag cgggacgcgg 60
   acgaaatcga acgagtgctc tccttcaccc acacc 95
<210> 20
   <211> 36
   <212> DNA
   <213> Artificial
<220>
   <223> primer-15
<400> 20
   ggcgcaattg cgtactttta cctaatcaac ccgttt 36
<210> 21
   <211> 33
   <212> DNA
   <213> Artificial
<220>
   <223> primer-16
<400> 21
   gaggcaattg cgtactttta cctaatcaac ccg 33
<210> 22
   <211> 38
   <212> DNA
   <213> Artificial
<220>
   <223> primer-17
<400> 22
   gtggcaattg cgaacgagcg tacttttacc taatcaac 38
<210> 23
   <211> 34
   <212> DNA
   <213> Artificial
<220>
   <223> primer-18
<400> 23
   tgttcaattg ccacgtagag ccagccaatg gcca 34
<210> 24
   <211> 90
   <212> DNA
   <213> Artificial
<220>
   <223> primer-19
<400> 24
   aacaatcgat gactttgtct ccttgaggtt atgttgcacc gcacagtata gcgggacgcg 60
   gacgaaatga tttgattgtc tctctgccgt 90
<210> 25
   <211> 71
   <212> DNA
   <213> Artificial
<220>
   <223> primer-20
<400> 25
   tttaggatcc cgcctcgggt gtgggtgaag gagagcacat gaaggaaatc ctggacgcga 60
   ttcaggccca g 71
<210> 26
   <211> 69
   <212> DNA
   <213> Artificial
<220>
   <223> primer-21
<400> 26
   gaagtggatc cttaaggcag cttgaccacg aatcctgcgt cagacgttgc ggaaacggtt 60
gatcgcgtc 69
<210> 27
   <211> 27
   <212> DNA
   <213> Artificial
<220>
   <223> primer-22
<400> 27
   gactagtccc gggcaagtac cttgccg 27
<210> 28
   <211> 32
   <212> DNA
   <213> Artificial
<220>
   <223> primer-23
<400> 28
   gactagtcgg ctgccgactg gttgaaccag gc 32
<210> 29
   <211> 1185
   <212> DNA
   <213> Cupriavidus necator
<400> 29
   atgacgcgtg aagtggtagt ggtaagcggt gtccgtaccg cgatcgggac ctttggcggc     60
   agcctgaagg atgtggcacc ggcggagctg ggcgcactgg tggtgcgcga ggcgctggcg    120
   cgcgcgcagg tgtcgggcga cgatgtcggc cacgtggtat tcggcaacgt gatccagacc    180
   gagccgcgcg acatgtatct gggccgcgtc gcggccgtca acggcggggt gacgatcaac    240
   gcccccgcgc tgaccgtgaa ccgcctgtgc ggctcgggcc tgcaggccat tgtcagcgcc    300
   gcgcagacca tcctgctggg cgataccgac gtcgccatcg gcggcggcgc ggaaagcatg    360
   agccgcgcac cgtacctggc gccggcagcg cgctggggcg cacgcatggg cgacgccggc    420
   ctggtcgaca tgatgctggg tgcgctgcac gatcccttcc atcgcatcca catgggcgtg    480
   accgccgaga atgtcgccaa ggaatacgac atctcgcgcg cgcagcagga cgaggccgcg    540
   ctggaatcgc accgccgcgc ttcggcagcg atcaaggccg gctacttcaa ggaccagatc    600
   gtcccggtgg tgagcaaggg ccgcaagggc gacgtgacct tcgacaccga cgagcacgtg    660
   cgccatgacg ccaccatcga cgacatgacc aagctcaggc cggtcttcgt caaggaaaac    720
   ggcacggtca cggccggcaa tgcctcgggc ctgaacgacg ccgccgccgc ggtggtgatg    780
   atggagcgcg ccgaagccga gcgccgcggc ctgaagccgc tggcccgcct ggtgtcgtac    840
   ggccatgccg gcgtggaccc gaaggccatg ggcatcggcc cggtgccggc gacgaagatc    900
   gcgctggagc gcgccggcct gcaggtgtcg gacctggacg tgatcgaagc caacgaagcc    960
   tttgccgcac aggcgtgcgc cgtgaccaag gcgctcggtc tggacccggc caaggttaac   1020
   ccgaacggct cgggcatctc gctgggccac ccgatcggcg ccaccggtgc cctgatcacg   1080
   gtgaaggcgc tgcatgagct gaaccgcgtg cagggccgct acgcgctggt gacgatgtgc   1140
   atcggcggcg ggcagggcat tgccgccatc ttcgagcgta tctga                   1185

## Description

### TECHNICAL FIELD

The present invention relates to a microorganism capable of producing a polyhydroxyalkanoate copolymer having a 3-hydroxyalkanoate unit with a carbon number of 4 or more, and a method for efficiently producing the copolymer using the microorganism.

### BACKGROUND ART

Polyhydroxyalkanoates (hereinafter, referred to as "PHAs") are polyester-type organic polymer molecules producible by various microorganisms. PHAs are thermoplastic polymers having biodegradability, and can be produced from recyclable resources. Therefore, there have43 been attempts to industrially produce PHAs as environment-conscious materials or biocompatible materials and utilize them in various industries.

Up to the present, many microorganisms are found to accumulate polyesters as energy storage materials in their cells. Typical examples of the polyesters include poly-3-hydroxybutyrate (hereinafter, also referred to as "P(3HB)"), which is a homopolymer of 3-hydroxybutyrate (hereinafter, also referred to as "3HB"). P(3HB) was first found in Bacillus megaterium in 1925. P(3HB) is a thermoplastic polymer and is biologically degraded in a natural environment. Thus, P(3HB) is focused on as an environmentally friendly plastic. However, P(3HB) is practically used in limited applications because it has high crystallinity and thereby is hard and brittle. For a wider range of applications, P(3HB) is required to have flexibility.

For this purpose, there is disclosed a method for producing a copolymer of 3HB and 3-hydroxyvalerate (hereinafter, referred to as "3HV") (hereinafter, this copolymer is referred to as P(3HB-co-3HV)) (Patent Documents 1 and 2). P(3HB-co-3HV) is a kind of PHA. P (3HB-co-3HV) has more flexibility than P(3HB), and thus has been expected to be used in a wide range of applications. Actually, however, the physical properties of P(3HB-co-3HV) are less likely to change when the molar fraction of 3HV therein is increased. In particular, this copolymer does not come to have flexibility enough to be processed into products such as films, sheets, and soft packaging containers. Thus, P(3HB-co-3HV) is only used in limited applications, hard molded products such as shampoo bottles and handles of throwaway razors.

In order to improve the flexibility of P(3HB), there has been performed studies about a copolymer of 3HB and 3-hydroxyhexanoate (hereinafter, also referred to as "3HH") (hereinafter, this copolymer is also referred to as P(3HB-co-3HH)) and a method for producing the same (Patent Documents 3 and 4). P(3HB-co-3HH) is also a kind of PHA. In the production method disclosed in these reports, P(3HB-co-3HH) is fermentatively produced by a wild-type strain of Aeromonas caviae isolated from soil with a fatty acid, such as oleic acid or palmitic acid, as a carbon source. The 3HH content was 15 mol% in the case that oleic acid was used as a carbon source, and 5 mol% in the case that palmitic acid was used as a carbon source.

There has also been performed studies about the physical properties of P(3HB-co-3HH) (Non-Patent Document 1). In this report, A. caviae was cultured with a fatty acid having 12 or more carbons as the only carbon source, and P(3HB-co-3HH) was fermentatively produced with the 3HH content of 11 to 19 mol%. This report shows that as the 3HH content was increased, P(3HB-co-3HH) gradually got flexible characteristics while losing hard and brittle characteristics like that of P(3HB), and finally exhibited flexibility higher than that of P(3HB-co-3HV). That is, P(3HB-co-3HH) can have a wide range of physical properties for applications from hard polymers to soft polymers according to the 3HH content therein. Thus, this copolymer is expected to be used in various applications from those requiring hardness, for example, TV housings produced from P(3HB-co-3HH) with a low 3HH content, to those requiring flexibility, for example, films produced from P(3HB-co-3HH) with a high 3HH content. However, the aforementioned production method gives low polymer productivity with a produced-cell amount of 4 g/L and a polymer content of 30% per cell. Hence, there has been demanded a method that gives higher productivity, especially a higher polymer content, for practical use.

There have been the following techniques as examples of efforts for industrial production of P(3HB-co-3HH). Non-Patent Document 2 discloses a technique in which Aeromonas hydrophila was fed-batch cultured for 43 hours with oleic acid as a carbon source to produce P(3HB-co-3HH) with a cell amount of 95.7 g/L, a polymer content of 45.2%, and a 3HH content of 17 mol%. There is disclosed another technique in which A. hydrophila was cultured with glucose and lauric acid as carbon sources, thereby achieving a 3HH content of 11 mol%, a cell amount of 50 g/L, and a polymer content of 50% (Non-Patent Document 3). However, A. hydrophila has pathogenicity to humans (Non-Patent Document 4), and thus is not suitable for industrial production. Further, the carbon sources used in these culture productions are expensive. Thus, an inexpensive carbon source is demanded in order to cut production cost.

Under such situations, the following efforts were performed for the purpose of producing this copolymer by a safe host and of improving productivity. A polyhydroxyalkanoate (PHA) synthase gene cloned from A. caviae was introduced into Cupriavidus necator (Old classification: Ralstonia eutropha or Alcaligenes eutrophus) to give a transformant, and this transformant was used with octanoic acid as a carbon source for production of P(3HB-co"3HH). As a result, this production method gave a 3HH content of 22 mol%, a cell amount of 4 g/L, and a polymer content of 33% by weight (Patent Document 5, Non-Patent Document 5). Further, the transformant was cultured with a vegetable oil or fat as a carbon source. As a result, this production method gave a 3HH content of 4 to 5 mol%, a cell amount of 4 g/L, and a polymer content of 80% (Non-Patent Document 6). The latter production method employed an inexpensive vegetable oil or fat as a carbon source and the polymer content was high; however, the cell amount was small and thus achieved low polymer productivity. In addition, the 3HH content of 4 to 5 mol% did not give flexibility sufficient for applications such as films.

There was also constructed a strain producing P(3HB-co-3HH) by using Escherichia coli as a host. That is, there was constructed a strain of E. coli in which genes such as an Aeromonas sp. PHA synthase gene and a NADP-acetoacetyl-CoA reductase gene of C. necator were introduced. The E. coli was cultured for 40.8 hours with dodecane as a carbon source, resulting in a cell amount of 79 g/L, a polymer content of 27.2%, and a 3HH content of 10.8 mol% (Non-Patent Document 8). There was also constructed E. coli into which an A. caviae PHA synthase gene, an enoyl-CoA hydratase gene, and an acyl-CoA dehydrogenase gene were introduced. The E. coli was cultured in a medium containing lauric acid, resulting in a cell productivity of 1 g/L, a polymer content of about 16%, and a 3HH content of about 16 mol% (Non-Patent Document 9). These E. coli strains also gave low polymer productivity, and it has been difficult to use these strains for industrial production.

In order to improve the productivity of P(3HB-co-3HH) and to control the 3HH content, PHA synthase was artificially modified (Non-Patent Document 10). This document reports that a mutant enzyme in which the 149th amino acid, asparagine, was substituted by serine and a mutant enzyme in which the 171st aspartic acid was substituted by glycine, among A. caviae-derived PHA synthase mutants, showed improved PHA synthase activity in E. coli and an increased 3HH content. The document further reports that a mutant enzyme in which the 518th phenylalanine was substituted by isoleucine and a mutant enzyme in which the 214th valine was substituted by glycine showed improved PHA synthase activity in E. coli and an increased polymer content. In these cases, however, a special E. coli strain was used as a host, and the polymer content was as low as about 13%. Thus, further improvement has been required for industrial production utilizing the characteristics of these mutant enzymes.

In another study, PHA synthase expression plasmids such as pJRDEE32 and pJRDEE32d13 (Patent Document 5, Non-Patent Document 5) were prepared by introducing genes such as a polyester synthase gene and an R-enoyl-CoA hydratase gene into pJRD215 (ATCC 37533). These plasmids were used to yield a transformant of C. necator, and the PHA productivity of this transformant was examined. The cell amount of this strain was as small as 4 g/L; however, in the case that the culture conditions of the strain with a vegetable oil or fat as a carbon source was changed for the better, the polymer productivity was improved to achieve a cell amount of 45 g/L, a polymer content of 62.5%, and a 3HH content of 8.1 mol%. As mentioned here, there were performed efforts for improving the 3HH content and the polymer productivity of P(3HB-co-3HH) by adjusting a culture method (Patent Document 6).

There is disclosed a method for controlling the physical properties of P(3HB-co-3HH) (Patent Document 6). Use of at least two of oils, fats and/or fatty acids having different carbon numbers as carbon sources enabled production of polyesters with a 3HH content of 1 to 40 mol%, and thereby enabled production of P(3HB--co-3HH) with various physical properties. This method, however, requires addition of a relatively expensive acid such as hexanoic acid or octanoic acid in order to control the 3HH content; here, high-concentration hexanoic acid shows cytotoxicity, resulting in lower cell productivity. Further, addition of multiple carbon sources may lead to complicated and high-cost production equipment.

There is also disclosed a method for increasing the 3HH content by using C. necator as a host and fructose as a carbon source for culture. In the case that a polyester synthase gene and a Streptomyces cinnamonensis-derived crotonyl-CoA reductase gene (hereinafter, abbreviated as "ccr") were introduced, the 3HH content was 0.9%; in the case that a gene such as an R-enoyl-CoA hydratase gene was further introduced, the 3HH content was increased to 1.6%. However, the cell amount was as small as about 1.5 g/L and the polymer content was as low as about 40% in this case. Thus, further improvement has been required for industrial production of P(3HB-co-3HH) with a high 3HH content (Non-Patent Document 7).

For applications such as films, sheets, and soft packaging containers, P(3HB-co-3HH) is desired to have a 3HH content of 12 mol% or higher. In the conventional P(3HB-co-3HH) production, however, attempts to increase the 3HH content resulted in reduction in the polymer content or the cell amount. There is no method achieving a polymer content of 70% or higher, a cell amount of 150 g/L or more, and a 3HH content of 12 mol% or higher, which are considered to be desirable values for industrial production. Thus, further improvement has been required in the production.
Patent Document 1: JP-A S57-150393
Patent Document 2: JP-A S59-220192
Patent Document 3: JP-A H05-93049
Patent Document 4: JP-A H07-265065
Patent Document 5: JP-A H10-108682
Patent Document 6: JP-A 2001-340078

Non-Patent Document 1: Y. Doi, S. Kitamura, H. Abe; Macromolecules, 28, 4822-4823 (1995)
Non-Patent Document 2: Biotechnology and Bioengineering, vol. 67, 240 (2000)
Non-Patent Document 3: Appl. Microbiol. Biotechnol., vol. 57, 50 (2001)
Non-Patent Document 4: Annex 1, Appendix 1, Byogentai tou Anzenkanri Kitei (biosafety manual) (1999), National Institute of Infectious Diseases (Japan)
Non-Patent Document 5: T. Fukui, Y. Doi; J. Bacteriol, 179, 15, 4821-4830 (1997)
Non-Patent Document 6: T. Fukui et al., Appl. Microbiol. Biotecnol., 49, 333 (1998)
Non-Patent Document 7: T. Fukui et al., Biomolecules, vol. 3, 618 (2002)
Non-Patent Document 8: S. Park et al., Biomacromolecules, vol. 2, 248 (2001)
Non-Patent Document 9: X. Lu et al., FEMS Microbiology Letters, vol. 221, 97 (2003)
Non-Patent Document 10: T. Kichise et al., Appl. Environ. Microbiol., 68, 2411-2419 (2002)

### SUMMARY OF THE INVENTION

An object of the present invention is to produce a PHA having a high 3HH content, which is excellent in flexibility and is expected to be used in various applications, by the use of an inexpensive vegetable oil as a carbon source at practical productivity. In particular, an object of the present invention is to effectively fermentation-produce P(3HB-co-3HH) having a 3HH content of 12 mol% or higher while maintaining a polymer content of 70% or higher in the cell and a cell amount of 150 g/L or more.

The present inventors have performed studies in order to solve the above problems; thereby, they have found that a large amount of PHA having an increased 3HH content can be produced and accumulated in a microorganism by increasing the PHA synthase amount, reducing the 3HB monomer-synthesizing ability, creating an additional 3HH monomer synthetic pathway, and enhancing the 3HH monomer-supplying system in the microorganism. Thus, the present inventors have completed the present invention.

A first aspect of the present invention relates to a microorganism capable of producing a polyhydroxyalkanoate having a 3-hydroxyhexanoate unit. In the microorganism, a phbA gene is inactivated, expressions of phaC and bktB β-ketothiolase) genes are enhanced, and a butyryl-CoA synthetic pathway is additionally introduced by ccr-gene introduction; thereby, the 3HH monomer-synthesizing ability is improved.

More specifically, the present invention relates to a microorganism satisfying the following requirements (1) to (3):
(1) expression of a phbA gene is repressed or a catalytic activity of an enzyme encoded by the gene is repressed;
(2) expression of a bktB gene is enhanced or a catalytic activity of an enzyme encoded by the gene is increased; and
(3) a polyhydroxyalkanoate synthase gene and a crotonyl-CoA reductase gene are introduced thereinto.

A second aspect of the present invention relates to a method for producing a 3HH unit-containing PHA having a 3HH content of 12 mol% or higher by the use of the above microorganism and an inexpensive carbon source while maintaining a polymer content of 70% or higher in the cell and a cell amount of 150 g/L or more.

The present invention enables fermentative production of a PHA having a 3HH content of 12 mol% or higher while maintaining a polymer content of 70% or higher in the cell and a cell amount of 150 g/L or more with an inexpensive and safe carbon source.

### BEST MODE FOR CARRYING OUT THE INVENTION

The following will describe the present invention in more detail. The 3HH unit-containing PHA herein means a polyester formed by copolymerization of 3HH and one or more 3-hydroxyalkanoates selected from the group consisting of 3HB, 3HV, 3-hydroxyoctanoate, and 3-hydroxyalkanoates having a longer alkyl chain than that of the foregoing.

The phbA gene herein means a gene coding for an enzyme that catalyzes condensation reaction between two molecules of acetyl-CoA but hardly catalyzes thiolysis of β-ketovaleryl-CoA among enzymes having β-ketothiolase activity. Examples thereof include a phbA gene having the base sequence under SEQ ID No. 5. The following gene can be suitably used in the present invention: a gene having a sequence identity of 85% or higher with the base sequence under SEQ ID No. 5 and coding for an enzyme that catalyzes condensation reaction between two molecules of acetyl-CoA but hardly catalyzes thiolysis of 3-ketovaleryl-CoA. The sequence identity is more preferably 90% or higher, further preferably 95% or higher, and still further preferably 98% or higher.

The bktB gene herein means a gene coding for an enzyme that well catalyzes both condensation reactions between two molecules of acetyl-CoA and between acetyl-CoA and an acyl-CoA having a longer chain length than that of the acetyl-CoA, such as propionyl-CoA, and catalyzes thiolysis of 3-ketovaleryl-CoA among enzymes having β-ketothiolase activity. Examples thereof include a gene having the base sequence under SEQ ID No. 29 (SLATER et al., J. Bacteriol., vol. 180, 1979-1987, 1998). The following gene can be suitably used in the present invention: a gene having a sequence identity of 85% or higher with the base sequence under SEQ ID No. 29 and coding for an enzyme that well catalyzes both condensation reactions between two molecules of acetyl-CoA and between acetyl-CoA and an acyl-CoA having a longer chain length than that of the acetyl-CoA, such as propionyl-CoA, and catalyzes thiolysis of 3-ketovaleryl-CoA. The sequence identity is more preferably 90% or higher, further preferably 95% or higher, and still further preferably 98% or higher.

Examples of the gene coding for a polymerase capable of synthesizing the 3HH unit-containing PHA include a phaC gene of A. caviae and a mutated phaC gene having the base sequence under SEQ ID No. 4. This gene can be any one that allows a host to produce the PHA. Suitably used in the present invention is a gene that has a sequence identity of 85% or higher with the base sequence of a phaC gene or the base sequence under SEQ ID No. 4, and codes for a polymerase capable of synthesizing the 3HH unit-containing PHA. The sequence identity is more preferably 90% or higher, further preferably 95% or higher, and still further preferably 98% or higher.

In the present invention, an endogenous promoter of the bktB structural gene is a DNA inducing transcription of the bktB structural gene. This promoter is endogenous to a microorganism that is used as a host and originally has the phbA gene and the bktB gene.

The microorganism originally having the phbA gene and the bktB gene used herein may be any wild-type strain having the phbA gene and the bktB gene or genetically manipulated microorganism derived from the wild-type strain. Any strain capable of synthesizing the 3HH unit-containing PHA can be used as a host. Specifically, preferable examples thereof include bacteria of Ralstonia, Cupriavidus, Wautersia, Aeromonas, Escherichia, Alcaligenes, and Pseudomonas species. More preferable are bacteria of Ralstonia, Cupriavidus, and Wautersia species for high safety and productivity. Particularly preferable is Cupriavidus necator. Examples of C. necator include C. necator H16 (ATCC 17699). This strain can be obtained from American Type Culture Collection (ATCC) and the like organizations. Of course, variants obtainable by artificial mutation of the aforementioned microorganisms and closely related strains mutated by a genetic engineering technique can be used in the present invention as long as they are capable of synthesizing the 3HH unit-containing PHA.

In order to increase the 3HH content, the phbA gene existing on the chromosome of the microorganism originally having the phbA gene and the bktB gene is repressed or inactivated. The enzyme encoded by the phbA gene catalyzes a reaction for producing acetoacetyl-CoA which is a precursor of 3-hydroxybutyryl-CoA, one of PEA monomers, by condensation of two acetyl-CoA molecules as mentioned above. On the other hand, the enzyme encoded by the phbA gene does not catalyze condensation reaction between acetyl-CoA and butanoyl-CoA. Thus, inactivation of the phbA gene causes reduction in the 3HB content in the polymer, presumably resulting in an increase in the 3HH content.

The method for inactivating the phbA gene may be any method as long as it finally inactivates phbA. Examples thereof include: 1) introduction of an additional termination codon between the initiation codon and the termination codon of the phbA gene; 2) introduction of a mutation that causes reduction in the ribosome-binding activity into the ribosomal binding site; 3) introduction of a mutation that causes reduction in the catalytic activity of the enzyme into the inside of the phbA structural gene; 4) utilization of RNA interference; 5) insertion of a transposon; and 6) deletion of part or all of the phbA structural gene. These methods are well known by a person skilled in the art.

In order to increase the 3HH content while increasing the PHA productivity, the enzyme encoded by the bktB gene is enhanced. The enzyme encoded by the bktB gene catalyzes not only the condensation reaction between two acetyl-CoA molecules but also the condensation reaction between acetyl-CoA and butanoyl-CoA. Condensation of acetyl-CoA and butanoyl-CoA yields 3-ketohexanoyl-CoA which is a precursor of 3-hydroxyhexanoyl-CoA. The enzyme encoded by the bktB gene has a higher activity for condensation of acetyl-CoA and butanoyl-CoA than for condensation of two acetyl-CoA molecules.

The enzyme encoded by the bktB gene may be enhanced by any method. High-level expression can be achieved using an expression vector or by alteration of an expression control region. Preferably, high-level expression can be achieved owing to an increase in transcriptional activity caused by altering a promoter of the gene on the chromosome of the microorganism originally having the phbA gene and the bktB gene, for example, by further inserting a promoter inducing transcription of the bktB gene into the upstream of the bktB gene endogenous to the microorganism originally having the phbA gene and the bktB gene.

The promoter inducing transcription of the bktB gene is preferably inserted into the upstream of the initiation codon of the bktB gene. Any promoter can be used in the present invention as long as it induces transcription of the bktB gene. The promoter is preferably at least either of a promoter endogenous to the microorganism originally having the phbA gene and the bktB gene or a promoter of a heterologous microorganism. For example, the promoter of phbCAB operon of C. necator having the base sequence under SEQ ID No. 1 and the promoter of phaPCJ operon of A. caviae having the base sequence under SEQ ID No. 2 are suitable in the case that C. necator is used as the microorganism originally having the phbA gene and the bktB gene.

The DNA used as the promoter preferably has a sequence identity of 70% or higher, more preferably 85% or higher, further preferably 90% or higher, still further preferably 95% or higher, and most preferably 98% or higher, with the base sequence under SEQ ID No. 1 or 2. Such a DNA can be used in the present invention as long as it induces transcription of the bktB gene.

Further, the DNA used as the promoter can be a DNA that hybridizes under a stringent condition in a hybridization process, such as colony hybridization, plaque hybridization, or Southern hybridization, with a base sequence complementary with the base sequence under SEQ ID No. 1 or 2 as a probe. Such a DNA can be used in the present invention as long as it induces transcription of the bktB gene.

The aforementioned hybridization can be performed in accordance with methods such as one disclosed in Molecular Cloning, A laboratory manual, second edition (Cold Spring Harbor Laboratory Press, 1989). Here, the DNA that hybridizes may be a DNA that can be obtained by the steps of: performing hybridization at 65°C in the presence of 0.7- to 1.0-M NaCl with a filter on which a colony- or plaque-derived DNA is immobilized; and then washing the filter with a double-concentrated SSC solution (it is noted that a 1-fold concentrated SSC solution contains 150-mM sodium chloride and 15-mM sodium citrate.) at 65°C. The DNA is preferably one obtainable by washing with a double-concentrated SSC solution at 65°C, more preferably washing with a 0.2-fold concentrated SSC solution at 65°C, and further preferably washing with a 0.1-fold concentrated SSC solution at 65°C.

Although the conditions for hybridization are mentioned above, the conditions are not limited to those mentioned. Conceivable factors that affect the stringency of hybridization may include multiple factors such as temperature and salt concentration, and a person skilled in the art will appropriately adjust the factors to achieve optimum stringency.

Examples of the DNA capable of hybridizing under the above conditions include those having a sequence identity of 70% or higher, preferably 85% or higher, more preferably 90% or higher, further preferably 95% or higher, and most preferably 98% or higher, with the DNA under SEQ ID No. 1 or 2. Such DNAs are included in the scope of the above promoter as long as they induce transcription of the bktB gene.

In order to further increase the 3HH content, it is effective to introduce a 3-hydroxybutyryl-CoA synthetic pathway by ccr-gene introduction. A crotonyl-CoA reductase encoded by the ccr gene is an enzyme that reduces crotonyl-CoA, an intermediate in the β-oxidation pathway of fatty acids, to produce butyryl-CoA, a substrate of the enzyme encoded by the bktB gene. The introduction of this gene not only results in direct supply of 3-hydroxyhexanoyl-CoA from the β-oxidation pathway but also in supply thereof from acetyl-CoA derived from the β-oxidation pathway.

Any ccr gene can be used in the present invention as long as it codes for an enzyme having an activity of reducing crotonyl-CoA to produce 3-hydroxyhexanoyl-CoA. The ccr gene is preferably a gene with a base sequence having a sequence identity of 70% or higher, more preferably 85% or higher, further preferably 90% or higher, still further preferably 95% or higher, and most preferably 98% or higher, with the base sequence under SEQ ID No. 3.

In addition, a DNA that hybridizes under a stringent condition in a hybridization process, such as colony hybridization, plaque hybridization, or Southern hybridization, with a base sequence complementary with the base sequence under SEQ ID No. 3 as a probe can be also used in the present invention as long as it codes for an enzyme having an activity of reducing crotonyl-CoA to produce butyryl-CoA.

Any polyhydroxyalkanoate synthase gene is used in the present invention as long as it codes for an enzyme having an activity of synthesizing a polyhydroxyalkanoate. It is preferably a gene having a base sequence with a sequence identity of 70% or higher, more preferably 85% or higher, further preferably 90% or higher, still further preferably 95% or higher, and most preferably 98% or higher, with the base sequence under SEQ ID No. 4.

Further, a DNA that hybridizes under a stringent condition in a hybridization process, such as colony hybridization, plaque hybridization, or Southern hybridization, with a base sequence complementary with the base sequence under SEQ ID No. 4 as a probe can be also used in the present invention as long as it codes for an enzyme having an activity of synthesizing a polyhydroxyalkanoate.

Examples of a carbon source that may be used for production of the 3HH-containing PHA include: saccharides such as glucose and fructose; alcohols such as methanol, ethanol, and butanol; fatty acids including saturated and unsaturated fatty acids such as acetic acid, propionic acid, hexanoic acid, octanoic acid, decanoic acid, lauric acid, oleic acid, palmitic acid, linolic acid, linolenic acid, and myristic acid; fatty acid derivatives such as esters and salts of the above fatty acids; organic acids such as lactic acid; fats and oils containing a large amount of saturated or unsaturated fatty acids having 10 or more carbons such as vegetable oils and fats, especially coconut oil, palm oil, palm kernel oil, palm kernel olein (hereinafter, also referred to as PKOO), and double fractionated palm olein (hereinafter, also referred to as POO). The amount of the carbon source upon culture is not particularly limited as long as it enables proliferation of the strain and synthesis of the polyester.

In the present invention, in the case that the wild-type strain of the microorganism originally having the phbA gene and the bktB gene used as a host hardly converts 3-hydroxyhexanoyl-CoA into a PHA, it is required to increase the efficiency of converting 3-hydroxyhexanoyl-CoA synthesized in the cells into a PHA. This increase may be achieved by inserting a gene coding for an enzyme that highly converts 3-hydroxyhexanoyl-CoA into a PHA in the chromosome or by introducing the same with a plasmid vector or the like. In the case that C. necator is used as a host, a phaC gene of A. caviae or a mutant gene thereof under SEQ ID No. 4 is preferably introduced as the gene coding for an enzyme that highly converts 3-hydroxyhexanoyl-CoA into a PHA.

The microorganism capable of producing the 3HH-containing PHA of the present invention may be prepared by any method. It may be prepared by the following method in which C. necator is used as a host. First, the original polyester synthase gene on the chromosome is substituted by a polyester synthase mutant gene which is derived from A. caviae and codes for an enzyme highly converting 3-hydroxyhexanoyl-CoA into a PHA by a technique such as homologous recombination. Then, the phbA gene existing on the chromosome is inactivated by a gene disruption technique. This inactivation can be achieved by any method as long as the activity of the protein produced by the phbA gene is reduced or eliminated; for example, a termination codon may be introduced into the inside of the phbA gene, or the promoter and/or ribosomal binding site at upstream of the gene may be altered. Further, expression of the bktB gene is enhanced. This enhancement can be achieved by any method as long as the activity of the product by the bktB gene is increased. For example, a bktB mutant gene having an improved specific activity may be used; a DNA having a heterologous or homologous promoter and ribosomal binding site may be inserted into the upstream of the initiation codon of the bktB gene; or the original promoter and/or ribosomal binding site of the bktB gene may be substituted by a DNA having a base sequence including a heterologous or homologous promoter and/or ribosomal binding site. The original promoter may be all eliminated from the chromosome, or may be partly deleted.

For the alteration of the chromosome, a method including site-specific insertion of or substitution by a gene in the chromosome is well known by a person skilled in the art. Examples of the typical method include, but not particularly limited to, the following methods: a method utilizing the mechanisms of transposon and homologous recombination (Ohman et al., J. Bacteriol., vol. 162, p1068 (1985)); a method based on principles of site-specific integration caused by the mechanism of homologous recombination, and elimination caused by the second homologous recombination event (Noti et al., Methods Enzymol., vol. 154, p197 (1987)); and in addition, a method in which a sacB gene derived from Bacillus subtilis is allowed to co-exist in a microorganism strain, and then the gene is eliminated by the second homologous recombination event, and thereby the microorganism strain is easily isolated as a strain resistant to a sucrose-added medium (Schweizer, Mol. Microbiol., vol. 6, p1195 (1992); Lenz et al., J. Bacteriol., vol. 176, p4385 (1994)). The alteration method is not particularly limited as long as insertion of or substitution by a gene in the chromosome is achieved.

The following will more specifically exemplify, for example, the method in which a DNA having a base sequence including the promoter and ribosomal binding site of a phaC gene of A. caviae is inserted just upstream of the initiation codon of the bktB gene in C. necator. First, a substitution fragment is prepared. The substitution fragment has a structure that the DNA having a base sequence including the promoter and ribosomal binding site of the phaC gene (hereinafter, also referred to as "phaC") of A. caviae is connected just upstream of the initiation codon of the bktB gene and followed by the bktB gene. That is, the substitution fragment is a DNA fragment in which the DNA having a base sequence including the promoter and ribosomal binding site of phaC is inserted just upstream of the initiation codon of the gene. The upstream and the downstream sequences of the DNA having a base sequence including the promoter and ribosomal binding site of phaC are homologous sequences required for homologous recombination with a DNA on the chromosome. In general, the longer the homologous sequence is, the more often the recombination occurs; here, the length may be freely set as long as it leads to homologous recombination.

To the substitution fragment may be added a gene that serves as a selective marker upon gene substitution. Examples of the gene that serves as a selective marker include genes resistant to antibiotics such as kanamycin, chloramphenicol, streptomycin, and ampicillin, and genes complementing various nutritional requirements. In the case that C. necator is used as a host, a kanamycin-resistant gene is suitable. In addition to these genes, to the fragment may be added a gene that makes it easy to select a microorganism strain in which a region including a selective marker gene is eliminated by the second homologous recombination event. Examples of such a gene include a sacB gene derived from Bacillus subtilis. It is known that a microorganism strain expressing this gene is incapable of growing on a sucrose-containing medium. Thus, such a strain in which this gene is eliminated can be easily selected out when grown on a sucrose-containing medium.

The substitution fragment having these sequences is connected to a vector that is not replicated in a host microorganism strain, and thereby prepared as a vector plasmid for gene substitution. Examples of such a plasmid which can be used for microorganisms such as bacteria of Ralstonia and Pseudomonas species include a pUC vector, a pBluescript vector, a pBR322 vector, and vectors having the same replication origin as that of those vectors. In addition, a DNA sequence that enables conjugal transfer, such as mob or oriT, may be allowed to co-exist.

The plasmid DNA for gene substitution prepared as having such a structure may be introduced into C. necator by a common method such as electroporation or conjugal transfer, and thereby homologous recombination can be caused.

Next, a strain with the plasmid DNA for gene substitution introduced on the chromosome by homologous recombination is selected out. The strain may be selected out by a method based on a selective gene co-existing with the plasmid DNA for gene substitution. If a kanamycin-resistant gene is used, the strain can be selected out from among strains grown on a kanamycin-containing medium.

In the next step, a strain in which a region including the selective marker gene has been eliminated from the chromosome by the second homologous recombination is selected out. Based on a selective gene used for the insertion, for example, a strain that has been made to be incapable of growing on a kanamycin-containing medium may be selected out; in the case that a sacB gene is allowed to co-exist with the plasmid for gene substitution, the target strain may be easily selected out from among strains grown on a sucrose-containing medium. Whether or not the thus-obtained strain is a strain in which genes are substituted as desired may be confirmed by known methods such as PCR, Southern hybridization, and determination of the DNA base sequence.

The aforementioned process provides a strain in which the DNA having a base sequence including the promoter and ribosomal binding site of phaC of A. caviae is inserted upstream of the initiation codon of the bktB structural gene on the chromosome in C. necator.

The following will describe an expression vector. Examples of the expression vector commonly used for C. necator include a pJRD215-derived expression vector (Patent Document 5, for example) and a pBBR122-derived expression vector (Non-Patent Document 7). The expression vector is desired to be stably replicated and maintained in host cells even under non-selective pressure conditions. Thus, vectors such as pCUP2 disclosed in WO/2007/049716 (see [0041]) are more suitable. The vector pCUP2 has a region required for replication and stable maintenance of a plasmid derived from the megaplasmid pMOL28 contained in Cupriavidus metallidurans CH34, which is closely related to C. necator.

A plasmid in which a phaC gene expression unit derived from A. caviae and a ccr gene expression unit derived from S. cinnamonensis are inserted into this pCUP2 is a pCUP2-631 vector. The pCUP2-631 vector may further have a phaP gene coding for a phasin derived from A. caviae and a phaJ gene coding for an R-specific enoyl-CoA hydratase derived from the same inserted thereinto. The pCUP2-631 vector may further have a phbP gene coding for a phasin derived from C. necator and a phaJ gene coding for an R-specific enoyl-CoA hydratase derived from A. caviae inserted thereinto.

In the case that this expression vector is introduced into the aforementioned strain in which the phbA gene on the chromosome is disrupted and expression of the bktB gene is enhanced, strains such as KNK-631 may be obtained, for example. These strains are capable of fermentation-producing a PHA having a 3HH content of 12 mol% or higher while maintaining a cell polymer content of 70% or higher and a cell amount of 150 g/L or more with an inexpensive and safe carbon source.

The following will describe the method for producing the 3HH-containing PHA with the microorganism prepared by the above method. The method is not particularly limited, and may be as follows. In the PHA production according to the present invention, the microorganism is preferably cultured in a medium containing a carbon source, nitrogen source, inorganic salts, and other organic nutrient sources.

Preferable examples of the carbon source include saccharides, fats and oils, and fatty acids, more preferably vegetable oils and fats, and further preferably palm. oil and palm kernel oil. Examples of the nitrogen source include ammonia, ammonium salts such as ammonium chloride, ammonium sulfate, and ammonium phosphate, peptone, meat extracts, and yeast extracts. Examples of the inorganic salts include potassium dihydrogenphosphate, sodium dihydrogenphosphate, magnesium phosphate, magnesium sulfate, and sodium chloride.

Examples of the other organic nutrient sources include amino acids such as glycine, alanine, serene, threonine, and proline, and vitamins such as vitamin B1, vitamin B12, and vitamin C. The culture solution may further contain antibiotics (e.g. kanamycin) corresponding to drug resistance genes existing in the expression plasmid.

In the present invention, the 3HH-containing PHA may be recovered from the cells by any method, and may be recovered as follows. As the culture has been finished, the cells are separated from the culture solution by a centrifuge or the like device; the cells are washed with distilled water, methanol or the like liquid, and then dried; the 3HH-containing PHA is extracted from these dried cells with an organic solvent such as chloroform; the cell components are removed from the resultant organic solvent containing the 3HH-containing PHA by filtration or the like process; a poor solvent such as methanol or hexane is added to this filtrate, and thereby the 3HH-containing PHA is precipitated; the supernatant fluid is removed by filtration, centrifugation, or the like process; and the residue was dried, and thereby the 3HH-containing PHA is recovered.

Analysis of the weight average molecular weight (Mw) and the 3HH content (mol%) of the obtained 3HH-containing PHA may be performed by techniques such as gas chromatography and nuclear magnetic resonance.

The highly flexible PHA obtained in the present invention is suitably used for products such as films, sheets, and soft packaging containers.

### EXAMPLES

The following will describe the present invention in more detail referring to, but not limited to, examples. Here, general gene manipulations may be performed as described in Molecular Cloning (Cold Spring Harbor Laboratory Press (1989)). Enzymes, cloning hosts, and the like materials used in gene manipulations may be those supplied by market providers, and should be used according to their instruction manuals. Enzymes are not particularly limited as long as they can be used in gene manipulations.

### (Example 1)

### <Preparation of plasmid vector for gene insertion>

A DNA having a base sequence including the promoter and ribosomal binding site of phaC of A. caviae was prepared as an insertion DNA as follows. PCR was performed with the genomic DNA of A. caviae as a template and primers under SEQ ID Nos. 6 and 7. Here, the PCR conditions were as follows: (1) 98°C for 2 minutes and (2) 98°C for 15 seconds, 60°C for 30 seconds, and 68°C for 20 seconds, repeated 25 cycles. The polymerase used here was KOD-plus-(TOYOBO Co., Ltd.). A DNA fragment obtained by PCR was terminally phosphorylated and was digested with EcoRI. This DNA fragment was called P_{Ac}-5P+Eco.

Next, the DNA insertion site was set as just upstream of the initiation codon of the bktB gene in the chromosomal DNA of the KNK-005AS strain disclosed in JP-A 2008-029218 (see [0038]), and a DNA having a base sequence upstream of the initiation codon of the gene was prepared as follows.

PCR was performed with the genomic DNA of the KNK-005 strain disclosed in JP-A 2008-029218 (see [0036]) as a template-DNA source and primers under SEQ ID Nos. 8 and 9. Thereby, a DNA having a base sequence upstream of the initiation codon of the bktB gene was prepared. Here, the PCR conditions were as follows: (1) 98°C for 2 minutes and (2) 98°C for 15 seconds, 64°C for 30 seconds, and 68°C for 30 seconds, repeated 25 cycles. The polymerase used here was KOD-plus-. The DNA fragment obtained by PCR was simultaneously digested with two restriction enzymes, BamHI and EcoRI. This DNA fragment was called P_{bktB}-Bam+Eco.

Then, P_{AC}-5P+Eco and P_{bktB}-Bam+Eco were ligated, and PCR was performed with a DNA generated in the ligation solution as a template DNA and primers under SEQ ID Nos. 8 and 7. Here, the PCR conditions were as follows: (1) 98°C for 2 minutes, 98°C for 15 seconds, 60°C for 30 seconds, and 68°C for 50 seconds, repeated 25 cycles. The polymerase used here was KOD-plus-. A DNA fragment obtained by PCR was terminally phosphorylated and was digested with BamHI. This DNA fragment was called bPac-5P+Bam.

Next, a DNA having a base sequence downstream of the initiation codon of the above gene was prepared. PCR was performed with the genomic DNA of the KNK-005 strain as a template-DNA source and primers under SEQ ID Nos. 10 and 11. Thereby, a DNA having a base sequence downstream of the initiation codon of the bktB gene was prepared. Here, the PCR conditions were as follows: (1) 98°C for 2 minutes and (2) 98°C for 15 seconds, 64°C for 30 seconds, and 68°C for 30 seconds, repeated 25 cycles. The polymerase used here was KOD-plus-. The DNA fragment obtained by PCR was terminally phosphorylated and was digested with ClaI. This DNA fragment was called ORF-5P+Cla.

Then, bPac-5P+Bam and ORF-5P+Cla were ligated, and PCR was performed with a DNA generated in the ligation solution as a template DNA and primers under SEQ ID Nos. 8 and 11. Here, the PCR conditions were as follows: (1) 98°C for 2 minutes and (2) 98°C for 15 seconds, 60°C for 30 seconds, and 68°C for 1 minute and 30 seconds, repeated 25 cycles. The polymerase used here was KOD-plus-. A DNA fragment obtained by PCR was simultaneously digested with two enzymes, BamHI and ClaI. This DNA fragment was subcloned into a vector pBluescript II KS (-) (TOYOBO Co., Ltd.) at the site digested with the same restriction enzymes. The obtained vector was called bAO/pBlu. The base sequence was determined with a DNA sequencer "3130x1 Genetic Analyzer" (APPLIED BIOSYSTEMS), and was confirmed to be the same as the base sequence of the template DNA.

Thereafter, pSACKm disclosed in JP-A 2008-029218 (see [0037]) was treated with a restriction enzyme NotI, and thereby an about 5.7-kb DNA fragment including a kanamycin-resistant gene and a sacB gene was cut out. This DNA fragment was inserted into bAO/pBlu at the site cleaved by the same enzyme. Thereby, a plasmid bAO/pBlu/SacB-Km for gene disruption and insertion was prepared.

### <Preparation of gene insertion strain Pac-bktB/AS>

The KNK-005AS strain, serving as a parent strain, and bAO/pBlu/SacB-Km were used to prepare a strain in which the DNA having a base sequence including the promoter and ribosomal binding site of phaC was inserted just upstream of the initiation codon of the bktB gene. E. coli S17-1 (ATCC 47005) was transformed with the gene insertion plasmid bAO/pBlu/SacB-Km to produce a transformant. The obtained transformant was co-cultured with the KNK-005AS strain on a Nutrient Agar medium (Difco) to cause conjugal transfer. A strain grown on a Simmons agar medium containing 250 mg/L of kanamycin (sodium citrate: 2 g/L, sodium chloride: 5 g/L, magnesium sulfate heptahydrate: 0.2 g/L, ammonium dihydrogenphosphate: 1 g/L, dipotassium hydrogenphosphate: 1 g/L, agar: 15 g/L, pH: 6.8) was selected out, and thereby the strain in which the plasmid was introduced in the chromosome of the KNK-005AS strain was obtained. This strain was cultured for two generations in a Nutrient Broth medium (Difco), and then diluted and plated on a 15% sucrose-containing Nutrient Agar medium. A strain grown was selected out, and thereby the strain in which the second recombination event occurred was obtained. Further, a desired gene-inserted strain was isolated by PCR analysis.

This gene-inserted strain was named Pac-bktB/AS. The base sequence was determined with a DNA sequencer "3130x1 Genetic Analyzer", and the strain was confirmed to be a strain in which the DNA having a base sequence including the promoter and ribosomal binding site of phaC was inserted just upstream of the initiation codon of the bktB gene.

### (Example 2) Preparation of Pre-bktB/AS strain

### <Preparation of plasmid vector for gene insertion>

A DNA having a base sequence including the promoter and ribosomal binding site of phbC of C. necator (hereinafter, referred to as "Pre") was prepared as an insertion DNA as follows. PCR was performed with the genomic DNA of the KNK-005 strain as a template and primers under SEQ ID Nos. 12 and 13. Here, the PCR conditions were as follows: (1) 98°C for 2 minutes and (2) 98°C for 15 seconds, 54°C for 30 seconds, and 68°C for 25 seconds, repeated 25 cycles. The polymerase used here was KOD-plus-. A DNA fragment obtained by PCR was terminally phosphorylated. This DNA fragment was called Pᵣₑ-5P.

Next, the DNA insertion site was set as just upstream of the initiation codon of the bktB gene in the chromosomal DNA of the KNK-005AS strain.

ORF-5P+Cla prepared in Example 1 was used as a DNA having a base sequence downstream of the initiation codon of the bktB gene.

Then, Pᵣₑ-5P and ORF-5P+Cla were ligated, and PCR was performed with a DNA generated in the ligation solution as a template DNA and primers under SEQ ID Nos. 12 and 11. Here, the PCR conditions were as follows: (1) 98°C for 2 minutes and (2) 98°C for 15 seconds, 54°C for 30 seconds, and 68°C for 50 seconds, repeated 25 cycles. The polymerase used here was KOD-plus-. A DNA fragment obtained by PCR was terminally phosphorylated and was digested with ClaI. This DNA fragment was called PRO-5P+Gla.

A sequence upstream of the initiation codon of the above gene was prepared. PCR was performed with the genomic DNA of the Kink-005 strain as a template-DNA source and primers under SEQ ID Nos. 8 and 14. Thereby, a DNA having a base sequence upstream of the initiation codon of the bktB gene was prepared. Here, the PCR conditions were as follows: (1) 98°C for 2 minutes and (2) 98°C for 15 seconds, 58°C for 30 seconds, and 68°C for 20 seconds, repeated 25 cycles. The polymerase used here was KOD-plus-The DNA fragment obtained by PCR was terminally phosphorylated and was digested with BamHI. This DNA fragment was called P_{bktB}-5P+Bam.

Then, P_{bktB}-5P+Bam and PRO-5P+Cla were ligated, and PCR was performed with a DNA generated in the ligation solution as a template DNA and primers under SEQ ID Nos. 9 and 11. Here, the PCR conditions were as follows: (1) 98°C for 2 minutes and (2) 98°C for 15 seconds, 60°C for 30 seconds, and 68°C for 1 minute and 30 seconds, repeated 25 cycles. The polymerase used here was KOD-plus-. A DNA fragment obtained by PCR was simultaneously digested with two enzymes, BamHI and ClaI. This DNA fragment was subcloned into a vector pBluescript II KS (-) at the site digested with the same restriction enzymes. The obtained vector was called bRO/pBlu. The base sequence was determined and was confirmed to be the same as the base sequence of the template DNA.

Thereafter, pSACKm was treated with a restriction enzyme NotI, and thereby an about 5.7-kb DNA fragment including a kanamycin-resistant gene and a sacB gene was cut out. This DNA fragment was inserted into bRO/pBlu at the site cleaved by the same enzyme. Thereby, a plasmid bRO/pBlu/SacB-Km for gene disruption and insertion was prepared.

### <Preparation of gene insertion strain Pre-bktB/AS>

In the same manner as the method for preparing a gene substitution strain in Example 1, the KNK-005AS strain, serving as a parent strain, and bRO/pBlu/SacB-Km were used to prepare a strain in which the DNA having a base sequence including the promoter and ribosomal binding site of phbC was inserted just upstream of the initiation codon of the bktB gene. This gene-inserted strain was named Pre-bktB/AS. The base sequence was determined and the strain was confirmed to be a strain in which the DNA having a base sequence including the promoter and ribosomal binding site of phbC was inserted just upstream of the initiation codon of the bktB gene.

### (Example 3) Preparation of BAB3/AS strain

### <Preparation of plasmid vector for gene insertion>

The DNA insertion site was set between the 91st and 92nd bases upstream from the initiation codon of the bktB gene in the chromosomal DNA of the KNK-005AS strain, in other words, just downstream of the termination codon of an ORF existing upstream of the bktB gene.

A DNA having a base sequence including the promoter and ribosomal binding site of phaPCJ of A. caviae was prepared as an insertion DNA as follows.

PCR was performed with the genomic DNA of A. caviae as a template and primers under SEQ ID Nos. 15 and 16. Here, the PCR conditions were as follows: (1) 98°C for 2 minutes and (2) 98°C for 15 seconds, 60°C for 30 seconds, and 68°C for 20 seconds, repeated 25 cycles. The polymerase used here was KOD-plus-. A DNA fragment obtained by PCR was digested with MunI. This DNA fragment was called P_{Ac}-Mun/3.

Next, a DNA having a base sequence upstream of the insertion site was prepared as follows.

PCR was performed with the genomic DNA of the KNK-005 strain as a template-DNA source and primers under SEQ ID Nos. 17 and 18. Thereby, a DNA having a base sequence of an ORF upstream of the bktB gene was prepared. Here, the PCR conditions were as follows: (1) 98°C for 2 minutes and (2) 98°C for 15 seconds, 64°C for 30 seconds, and 68°C for 30 seconds, repeated 25 cycles. The polymerase used here was KOD-plus-. The DNA fragment obtained by PCR was digested with a restriction enzyme MunI. This DNA fragment was called miaB-Mun/3.

Then, P_{Ac}-Mun/3 and miaB-Mun/3 were ligated, and PCR was performed with a DNA generated in the ligation solution as a template DNA and primers under SEQ ID Nos. 17 and 16. Here, the PCR conditions were as follows: (1) 98°C for 2 minutes and (2) 98°C for 15 seconds, 60°C for 30 seconds, and 68°C for 60 seconds, repeated 25 cycles. The polymerase used here was KOD-plus-. A DNA fragment obtained by PCR was digested with BamHI and ClaI. This DNA fragment was called BAB3-Bam+Cla.

This DNA fragment was subcloned into a vector pBluescript II KS (-) at the site digested with the same restriction enzymes. The obtained vector was called BAB3/pBlu. The base sequence was determined, and was confirmed to be a desired base sequence.

Thereafter, pSACKm was treated with a restriction enzyme NotI, and thereby an about 5.7-kb DNA fragment including a kanamycin-resistant gene and a sacB gene was cut out. This DNA fragment was inserted into BAB3/pBlu at the site cleaved by the same enzyme. Thereby, a plasmid BAB3/pBlu/SacB-Km for gene disruption and insertion was prepared.

### <Preparation of gene insertion strain BAB3/AS>

In the same manner as the method for preparing a gene insertion strain in Example 1, the KNK-005AS strain, serving as a parent strain, and BAB3/pBlu/SacB-Km were used to prepare a strain in which the DNA having a base sequence including the promoter and ribosomal binding site of phaPCJ was inserted upstream of the initiation codon of the bktB structural gene. This DNA-inserted strain was named BAB3/AS. The base sequence was determined, and the strain was confirmed to be a strain in which the DNA having a base sequence including the promoter and ribosomal binding site of phaPCJ was inserted between the 91st and 92nd bases upstream from the initiation codon of the bktB gene.

### (Example 4) Preparation of BAB4/AS strain

### <Preparation of plasmid vector for gene insertion>

The DNA insertion site was set between the 65th and 66th bases upstream from the initiation codon of the bktB gene in the chromosomal DNA of the KNK-005AS strain, in other words, 26 bases downstream from the termination codon of an ORF existing upstream of the bktB gene.

A DNA having a base sequence including the promoter and ribosomal binding site of phaPCJ of A. caviae was prepared as an insertion DNA as follows.

PCR was performed with the genomic DNA of A. caviae as a template and primers under SEQ ID Nos. 15 and 19. Here, the PCR conditions were as follows: (1) 98°C for 2 minutes and (2) 98°C for 15 seconds, 60°C for 30 seconds, and 68°C for 20 seconds, repeated 25 cycles. The polymerase used here was KOD-plus-. A DNA fragment obtained by PCR was digested with MunI. This DNA fragment was called P_{Ac}-Mun/4.

Next, a DNA having a base sequence upstream of the insertion site was prepared as follows.

PCR was performed with the genomic DNA of the KNK-005 strain as a template-DNA source and primers under SEQ ID Nos. 17 and 20. Thereby, a DNA having a base sequence of an ORF upstream of the bktB gene was prepared. Here, the PCR conditions were as follows: (1) 98°C for 2 minutes and (2) 98°C for 15 seconds, 64°C for 30 seconds, and 68°C for 30 seconds, repeated 25 cycles. The polymerase used here was KOD-plus-. The DNA fragment obtained by PCR was digested with a restriction enzyme MunI. This DNA fragment was called miaB-Mun/4.

Then, P_{Ac}-Mun/4 and miaB-Mun/4 were ligated, and PCR was performed with a DNA generated in the ligation solution as a template DNA and primers under SEQ ID Nos. 17 and 19. Here, the PCR conditions were as follows: (1) 98°C for 2 minutes and (2) 98°C for 15 seconds, 60°C for 30 seconds, and 68°C for 60 seconds, repeated 25 cycles. The polymerase used here was KOD-plus-. A DNA fragment obtained by PCR was digested with BamHI and ClaI. This DNA fragment was called BAB4-Bam+Cla.

This DNA fragment was subcloned into a vector pBluescript II KS (-) at the site digested with the same restriction enzymes. The obtained vector was called BAB4/pBlu. The base sequence was determined, and was confirmed to be a desired base sequence.

Thereafter, pSACKm was treated with a restriction enzyme NotI, and thereby an about 5.7-kb DNA fragment including a kanamycin-resistant gene and a sacB gene was cut out. This DNA fragment was inserted into BAB4/pBlu at the site cleaved by the same enzyme. Thereby, a plasmid BAB4/pBlu/SacB-Km for gene disruption and insertion was prepared.

### <Preparation of gene insertion strain BAB4/AS>

In the same manner as the method for preparing a gene insertion strain in Example 1, the KNK-005AS strain, serving as a parent strain, and BAB4/pBlu/SacB-Km were used to prepare a strain in which the DNA having a base sequence including the promoter and ribosomal binding site of phaPCJ was inserted upstream of the initiation codon of the bktB structural gene. This DNA-inserted strain was named BAB4/AS. The base sequence was determined, and the strain was confirmed to be a strain in which the DNA having a base sequence including the promoter and ribosomal binding site of phaPCJ was inserted between the 65th and 66th bases upstream from the initiation codon of the bktB structural gene.

### (Example 5) Preparation of BAB5/AS strain

### <Preparation of plasmid vector for gene insertion>

The DNA insertion site was set between the 58th and 59th bases upstream from the initiation codon of the bktB gene in the chromosomal DNA of the KNK-005AS strain, in other words, 33 bases downstream from the termination codon of an ORF existing upstream of the bktB gene.

A DNA having a base sequence including the promoter and ribosomal binding site of phaPCJ of A. caviae was prepared as an insertion DNA as follows.

PCR was performed with the genomic DNA of A. caviae as a template and primers under SEQ ID Nos. 15 and 21. Here, the PCR conditions were as follows: (1) 98°C for 2 minutes and (2) 98°C for 15 seconds, 60°C for 30 seconds, and 68°C for 20 seconds, repeated 25 cycles. The polymerase used here was KOD-plus-. A DNA fragment obtained by PCR was digested with MunI. This DNA fragment was called P_{Ac}-Mun/5.

Next, a DNA having a base sequence upstream of the insertion site was prepared as follows.

PCR was performed with the genomic DNA of the KNK-005 strain as a template-DNA source and primers under SEQ ID Nos. 17 and 22. Thereby, a DNA having a base sequence of an ORF upstream of the bktB gene was prepared. Here, the PCR conditions were as follows: (1) 98°C for 2 minutes and (2) 98°C for 15 seconds, 64°C for 30 seconds, and 68°C for 30 seconds, repeated 25 cycles. The polymerase used here was KOD-plus-. The DNA fragment obtained by PCR was digested with a restriction enzyme MunI. This DNA fragment was called miaB-Mun/5.

Then, P_{Ac}-Mun/5 and miaB-Mun/5 were ligated, and PCR was performed with a DNA generated in the ligation solution as a template DNA and primers under SEQ ID Nos. 17 and 21. Here, the PCR conditions were as follows: (1) 98°C for 2 minutes and (2) 98°C for 15 seconds, 60°C for 30 seconds, and 68°C for 60 seconds, repeated 25 cycles. The polymerase used here was KOD-plus-. A DNA fragment obtained by PCR was digested with BamHI and Clal. This DNA fragment was called BAB5-Bam+Cla.

This DNA fragment was subcloned into a vector pBluescript II KS (-) at the site digested with the same restriction enzymes. The obtained vector was called BAB5/pBlu. The base sequence was determined, and the DNA was confirmed to have a desired base sequence.

Thereafter, pSACKm was treated with a restriction enzyme NotI, and thereby an about 5.7-kb DNA fragment including a kanamycin-resistant gene and a sacB gene was cut out. This DNA fragment was inserted into BAB5/pBlu at the site cleaved by the same enzyme. Thereby, a plasmid BAB5/pBlu/SacB-Km for gene disruption and insertion was prepared.

### <Preparation of gene insertion strain BAB5/AS>

In the same manner as the method for preparing a gene insertion strain in Example 1, the KNK-005AS strain, serving as a parent strain, and BAB5/pBlu/SacB-Km were used to prepare a strain in which the DNA having a base sequence including the promoter and ribosomal binding site of phaPCJ was inserted upstream of the initiation codon of the bktB gene. This DNA-inserted strain was named BAB5/AS. The base sequence was determined, and the strain was confirmed to be a strain in which the DNA having a base sequence including the promoter and ribosomal binding site of phaPCJ was inserted between the 58th and 59th bases upstream from the initiation codon of the bktB gene.

### (Example 6) Preparation of BRB5/AS strain

### <Preparation of plasmid vector for gene insertion>

The DNA insertion site was set between the 58th and 59th bases upstream from the initiation codon of the bktB gene in the chromosomal DNA of the KNK-005AS strain, in other words, 33 bases downstream from the termination codon of an ORF existing upstream of the bktB gene.

A DNA having a base sequence including the promoter and ribosomal binding site of phbC of C. necator was prepared as an insertion DNA as follows.

PCR was performed with the genomic DNA of C. necator as a template and primers under SEQ ID Nos. 23 and 24. Here, the PCR conditions were as follows: (1) 98°C for 2 minutes and (2) 98°C for 15 seconds, 60°C for 30 seconds, and 68°C for 20 seconds, repeated 25 cycles. The polymerase used here was KOD-plus-. A DNA fragment obtained by PCR was digested with a restriction enzyme MunI. This DNA fragment was called P_{Re}-Mun/5.

Then, P_{Re}-Mun/5 and miaB-Mun/5 prepared in Example 5 were ligated, and PCR was performed with a DNA generated in the ligation solution as a template DNA and primers under SEQ ID Nos. 17 and 24. Here, the PCR conditions were as follows: (1) 98°C for 2 minutes, 98°C for 15 seconds, 60°C for 30 seconds, and 68°C for 60 seconds, repeated 25 cycles. The polymerase used here was KOD-plus-. A DNA fragment obtained by PCR was digested with BamHI and ClaI. This DNA fragment was called BRB5-Bam+Cla.

This DNA fragment was subcloned into a vector pBluescript II KS (-) at the site digested with the same restriction enzymes. The obtained vector was called BRB5/pBlu. The base sequence was determined, and the DNA was confirmed to have a desired base sequence.

Thereafter, pSACKm was treated with a restriction enzyme NotI, and thereby an about 5.7-kb DNA fragment including a kanamycin-resistant gene and a sacB gene was cut out. This DNA fragment was inserted into BRB5/pBlu at the site cleaved by the same enzyme. Thereby, a plasmid BRB5/pBlu/SacB-Km for gene disruption and insertion was prepared.

### <Preparation of gene insertion strain BRB5/AS>

In the same manner as the method for preparing a gene insertion strain in Example 1, the KNK-005AS strain, serving as a parent strain, and BRB5/pBlu/SacB-Km were used to prepare a strain in which the DNA having a base sequence including the promoter and ribosomal binding site of phbC was inserted upstream of the initiation codon of the bktB gene. This DNA-inserted strain was named BRB5/AS. The base sequence was determined, and the strain was confirmed to be a strain in which the DNA having a base sequence including the promoter and ribosomal binding site of phbC was inserted between the 58th and 59th bases upstream from the initiation codon of the bktB structural gene.

### (Example 7)

### <Cloning of ccr gene and construction of expression unit>

A ccr gene coding for an enzyme that converts crotonyl-CoA into butyryl-CoA, a precursor of the 3HH monomer, was cloned from the chromosomal DNA of Streptomyces cinnamonensis Okami (DSM 1042). PCR was performed with DNAs under SEQ ID Nos. 25 and 26 as primers. The PCR conditions were as follows: (1) 98°C for 2 minutes and (2) 94°C for 10 seconds, 55°C for 20 seconds, and 68°C for 90 seconds, repeated 25 cycles. The polymerase used here was KOD-plus-. Fragments amplified by PCR were purified, and then cleaved with restriction enzymes BamHI and AflII. An EE32d13 fragment (J. Bacteriol., 179, 4821 (1997)) was subcloned into the EcoRI site of a pUC19 vector, and this plasmid was cleaved with BglII and AflII. The cleaved site of the plasmid was substituted by the BamHI-AflII fragment of the ccr gene, and thereby a ccr expression unit was constructed.

### (Example 8)

### <Cloning of phaC gene and preparation of expression unit>

A phaC expression unit having the sequence under SEQ ID No. 4 was prepared as an SpeI fragment. PCR was performed with HG::P_{Re}-N149S/D171G-T/pBlu disclosed in JP-A 2007-228894 (see [0031]) as a template and primers under SEQ ID Nos. 27 and 28. The PCR conditions were as follows: (1) 98°C for 2 minutes and (2) 94°C for 10 seconds, 55°C for 30 seconds, and 68°C for 2 minutes, repeated 25 cycles. The polymerase used here was KOD-plus-. Fragments amplified by PCR were purified and cleaved by a restriction enzyme SpeI, and thereby the expression unit was prepared.

### (Example 9)

### <Construction of expression vector>

An expression vector pCUP2-631 was constructed as follows.

The vector pCUP22 disclosed in WO/2007/049716 (see [0041]) was used as a plasmid vector for constructing an expression vector in C. necator. First, the ccr gene expression unit constructed in Example 7 was cut out with EcoRI, and this fragment was ligated with pCUP2 cleaved with MunI. Then, the phaC expression unit prepared in Example 8 was prepared as an SpeI fragment, and inserted into the SpeI site of the pCUP2 including the ccr gene expression unit. Thereby, a pCUP2-631 vector was constructed.

### (Example 10) Preparation of transformed cell

The pCUP2-631 vector was electrically introduced into various cells as follows. The gene introducer used here was Gene Pulser (BioRad), and the cuvette was one having a gap of 0.2 cm (also Biorad). The cuvette was charged with 400 µl of competent cells and 20 µl of the expression vector, and placed on the pulser. Electric pulses were applied thereto at a capacitance of 25 µF, a voltage of 1.5 kV, and a resistance of 800 Ω. After pulse application, the bacterial suspension in the cuvette was shake-cultured at 30°C for 3 hours in a Nutrient Broth medium (DIFCO) and was cultured at 30°C for 2 days on a selective plate (Nutrient Agar medium (DIFCO), kanamycin: 100 mg/L). Then, a growing transformant was obtained.

### (Example 11)

Various transformants prepared were cultured. The pre-medium contained 1%(w/v) Meat-extract, 1%(w/v) Bacto-Trypton, 0.2%(w/v) Yeast-extract, 0.9%(w/v) Na₂HPO₄·12H₂O, and 0.15%(w/v) KH₂PO₄, and had a pH of 6.7.

The polyester-producing medium contained 1.1%(w/v) Na₂HPO₄·12H₂O, 0.19%(w/v) KH₂PO₄, 0.6%(w/v) (NH₄)₂SO₄, 0.1%(w/v) MgSO₄·7H₂O, and 0.5%(v/v) trace mineral salt solution (1.6%(w/v) FeCl₃·6E₂O, 1%(w/v) CaCl₂·2H₂O, 0.02%(w/v) CoCl₂·6H₂O, 0.016%(w/v) CuSo₄·5H₂O, 0.012%(w/v) NiCl₂·6H₂O, and 0.01%(w/v) CrCl₃·6B₂O dissolved in 0.1 N hydrochloric acid). Here, the culture was performed by fed-batch culture with PKOO (palm kernel olein fraction) fed as a carbon source.

A glycerol stock of each transformant was inoculated in the pre-medium and cultured for 20 hours. Then, 10%(v/v) of the resultant culture was inoculated in a 5-L jar fermentor (model MD-500, B. E. Marubishi Co., Ltd.) containing 2.5 L of the polyester-producing medium. The fermentor was operated at a culturing temperature of 28°C, a stirring rate of 420 rpm, and a ventilation amount of 0.6 vvm, and the pH was controlled between 6.6 and 6.8. The pH was controlled with 14% aqueous ammonia. The culture was performed for 65 hours. The cells were recovered by centrifugation after the culture. Then, the cells were washed with methanol and freeze-dried. Thereafter, the 3HH content was analyzed.

The 3HH content of the produced polyester was analyzed by gas chromatography as follows. About 20 mg of the dried polyester was added with 2 ml of a mixed solution of sulfuric acid and methanol (15:85) and 2 ml of chloroform. The mixture was sealed and heated at 100°C for 140 minutes. Thereby, a methyl ester of the decomposed polyester was obtained. This reaction mixture was cooled down, and then 1.5 g of sodium hydrogencarbonate was gradually added thereto so as to neutralize the mixture. The resultant mixture was left standing until generation of carbon dioxide gas stopped. The mixture was sufficiently mixed with 4 ml of diisopropyl ether, and then centrifuged. The monomer unit composition of the decomposed polyester in the supernatant was analyzed by capillary gas chromatography. The gas chromatograph used here was GC-17A (Shimadzu Corp.), and the capillary column was NEUTRABOND-1 (column length: 25 m, column inner diameter: 0.25 mm, liquid film thickness: 0.4 µm, GL Sciences, Inc.). The carrier gas was He, the column inlet pressure was 100 kPa, and the amount of the sample injected was 1 µl. The temperature was increased at a rate of 8°C/min from an initial temperature of 100°C to 200°C and was then increased at rate of 30°C/min from 200°C to 290°C. As a result of analysis under the above conditions, each of the polyesters was a copolymer polyester P(3HB-co-3HH) as shown in formula (1). Table 1 shows the dry cell amount, polymer content, and 3HH content.

**[Table 1]**

| Host+vector | Dry cell amount (g/L) | Polymer content (%) | 3HH content (mol%) |
|---|---|---|---|
| Pac-bktB/AS+pCUP2-631 | 159 | 79 | 13.5 |
| Pre-bktB/AS+pCUP2-631 | 172 | 79 | 11.0 |
| BAB3/AS+pCUP2-631 | 150 | 75 | 14.0 |
| BAB4/AS+pCUP2-631 | 173 | 83 | 7.9 |
| BAB5/AS+pCUP2-631 | 161 | 80 | 14.3 |
| BRB5/AS+pCUP2-631 | 156 | 71 | 24.3 |

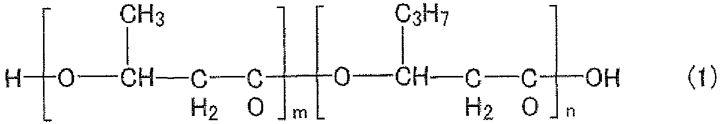

In the formula, m and n each represent an integer of 1 or greater.

## Claims

1. A microorganism, satisfying the following requirements (1) to (3):
(1) expression of a phbA gene is repressed or a catalytic activity of an enzyme encoded by the gene is repressed;
(2) expression of a bktB gene is enhanced or a catalytic activity of an enzyme encoded by the gene is increased; and
(3) a polyhydroxyalkanoate synthase gene and a crotonyl-CoA reductase gene are introduced thereinto.

2. The microorganism according to claim 1,
wherein the expression of the phbA gene is repressed or the catalytic activity of the enzyme encoded by the gene is repressed, by at least one of the following manipulations (1) to (6):
(1) introduction of an additional termination codon between the initiation codon and the termination codon of the phbA gene;
(2) introduction of a mutation that causes reduction in a ribosome-binding activity into a ribosomal binding site of the phbA gene;
(3) introduction of a mutation that causes reduction in the catalytic activity of the enzyme encoded by the phbA gene into the inside of the phbA gene;
(4) utilization of RNA interference;
(5) insertion of a transposon into the inside of the phbA gene; and
(6) deletion of part or all of the phbA gene.

3. The microorganism according to claim 1,
wherein the expression of the bktB gene is enhanced by additional insertion of a promoter having an activity of inducing transcription of the bktB gene into upstream of the bktB gene.

4. The microorganism according to claim 3,
wherein the additionally inserted promoter is the following DNA (1) or (2):
(1) a DNA that, upstream of the bktB gene, has a sequence identity of 70% or higher with a DNA of the base sequence under SEQ ID No. 1 or 2, and has an activity of inducing transcription of the bktB gene; and
(2) a DNA that, upstream of the bktB gene, hybridizes with a DNA complementary with the DNA of the base sequence under SEQ ID No. 1 or 2 under a stringent condition, and has an activity of inducing transcription of the bktB gene.

5. The microorganism according to claim 1,
wherein at least one of the following polyhydroxyalkanoate synthase genes and/or crotonyl-CoA reductase genes (1) to (4) is introduced:
(1) a gene that has a sequence identity of 70% or higher with a DNA of the base sequence under SEQ ID No. 3, and codes for an enzyme having an activity of reducing crotonyl-CoA to produce butyryl-CoA;
(2) a gene that hybridizes with a DNA complementary with the DNA of the base sequence under SEQ ID No. 3 under a stringent condition, and codes for an enzyme having an activity of reducing crotonyl-CoA to produce butyryl-CoA;
(3) a gene that has a sequence identity of 70% or higher with a DNA of the base sequence under SEQ ID No. 4, and codes for an enzyme having an activity of synthesizing a polyhydroxyalkanoate; and
(4) a gene that hybridizes with a DNA complementary with the DNA of the base sequence under SEQ ID No. 4 under a stringent condition, and codes for an enzyme having an activity of synthesizing a polyhydroxyalkanoate.

6. The microorganism according to claim 5, which is produced by gene recombination.

7. The microorganism according to any one of claims 1 to 5, which is capable of producing a polyhydroxyalkanoate by use of a vegetable oil or fat as a carbon source with achieving a dry cell amount of 150 g/L or more, a polymer content of 70% or higher, and a 3-hydroxyhexanoate content of 12 mol% or higher.

8. The microorganism according to any one of claims 1 to 7,
wherein the polyhydroxyalkanoate is a polyester having 3-hydroxybutyrate and 3-hydroxyhexanoate as structural units.

9. The microorganism according to any one of claims 1 to 8, which is derived from Cupriavidus necator.

10. The microorganism according to claim 9, which is derived from Cupriavidus necator H 16.

11. A method for producing a polyhydroxyalkanoate having a 3-hydroxyhexanoate unit, the method comprising:
cultivating the microorganism according to any one of claims 1 to 10 with a vegetable oil or fat as a carbon source.

## Patentansprüche

1. Mikroorganismus, der die folgenden Voraussetzungen (1) bis (3) erfüllt:
(1) Die Expression eines phbA-Gens ist unterdrückt oder eine katalytische Aktivität eines Enzyms, das von diesem Gen kodiert wird, ist unterdrückt;
(2) die Expression eines bktB-Gens ist erhöht oder eine katalytische Aktivität eines Enzyms, das von diesem Gen kodiert wird, ist erhöht; und
(3) ein Polyhydroxyalkanoat-Synthase-Gen und ein Crotonyl-CoA-Reduktase-Gen sind darin eingefügt.

2. Mikroorganismus nach Anspruch 1,
wobei die Expression des phbA-Gens unterdrückt ist oder die katalytische Aktivität des Enzyms, das von diesem Gen kodiert wird, unterdrückt ist mittels mindestens einer der folgenden Manipulationen (1) bis (6);
(1) Einfügen eines zusätzlichen Schluss-Codons zwischen dem Anfangs-Codon und dem Schluss-Codon des phbA-Gens;
(2) Einfügen einer Mutation, die die Reduktion einer Ribosomen-Bindungsaktivität zur Folge hat, in eine ribosomale Bindestelle des phbA-Gens;
(3) Einfügen einer Mutation, die die Reduktion der katalytischen Aktivität des Enzyms, das von dem phbA-Gen kodiert wird, reduziert in das Innere des phbA-Gens;
(4) Verwendung von RNA-Interferenz;
(5) Insertion eines Transposons in das Innere des phbA-Gens; und
(6) Deletion eines Teils des phbA-Gens oder des gesamten phbA-Gens.

3. Mikroorganismus nach Anspruch 1,
wobei die Expression des bktB-Gens erhöht ist durch die zusätzliche Insertion eines Promotors, der eine Aktivität hat, die Transkription des bktB-Gens zu induzieren, stromaufwärts des bktB-Gens.

4. Mikroorganismus nach Anspruch 3,
wobei der zusätzlich eingefügte Promotor die folgende DNA (1) oder (2) ist:
(1) eine DNA, die stromaufwärts vom bktB-Gen eine Sequenzidentität von 70% oder höher mit einer DNA der Basissequenz nach SEQ ID No. 1 oder 2 hat und die eine Aktivität hat, die Transkription des bktB-Gens zu induzieren; und
(2) eine DNA, die stromaufwärts vom bktB-Gen mit einer DNA, die komplementär zu der DNA der Basissequenz nach SEQ ID No. 1 oder 2 ist, unter stringenten Bedingungen hybridisiert und eine Aktivität hat, die Transkription des bktB-Gens zu induzieren.

5. Mikroorganismus nach Anspruch 1,
wobei mindestens eines der folgenden Polyhydroxyalkanoat-Synthase-Gene und/oder Crotonyl-CoA-Reduktase-Gene (1) bis (4) eingefügt ist:
(1) ein Gen, das eine Sequenzidentität von 70% oder höher mit einer DNA der Basissequenz nach SEQ ID No. 3 hat und ein Enzym kodiert, das die Aktivität hat, Crotonyl-CoA zu reduzieren, um Butyryl-CoA herzustellen;
(2) ein Gen, das unter stringenten Bedingungen an eine DNA hybridisiert, die komplementär zu der DNA der Basissequenz nach SEQ ID No. 3 ist und ein Enzym kodiert, das die Aktivität hat, Crotonyl-CoA zu reduzieren, um Butyryl-CoA herzustellen;
(3) ein Gen, das eine Sequenzidentität von 70% oder höher mit einer DNA der Basissequenz nach SEQ ID No. 4 hat und ein Enzym kodiert, das die Aktivität hat, Polyhydroxyalkanoat zu synthetisieren;
(4) ein Gen, das unter stringenten Bedingungen an eine DNA hybridisiert, die komplementär zu der DNA der Basissequenz nach SEQ ID No. 4 ist und ein Enzym kodiert, das die Aktivität hat, Polyhydroxyalkanoat zu synthetisieren.

6. Mikroorganismus nach Anspruch 5, der durch Genrekombination hergestellt ist.

7. Mikroorganismus nach einem der Ansprüche 1 bis 5, der ein Polyhydroxyalkanoat herstellen kann unter Verwendung von Pflanzenöl oder -fett als Kohlenstoffquelle unter Erreichen einer Trockenzellmenge von 150g/L oder mehr, eines Polymer-Gehalts von 70% oder mehr und eines 3-Hydroxyhexanoat-Gehalts von 12 mol% oder mehr.

8. Mikroorganismus nach einem der Ansprüche 1 bis 7, wobei das Polyhydroxyalkanoat ein Polyester ist, der 3-Hydroxybutyrat und 3-Hydroxyhexanoat als Struktureinheiten hat.

9. Mikroorganismus nach einem der Ansprüche 1 bis 8, der von Cupriavidus necator abstammt.

10. Mikroorganismus nach Anspruch 9, der von Cupriavidus necator H16 abstammt.

11. Verfahren zur Herstellung eines Polyhydroxyalkanoats, das eine 3-Hydroxyhexanoat-Einheit hat, wobei das Verfahren umfasst:
Züchtung des Mikroorganismus nach einem der Ansprüche 1 bis 10 mit einem Pflanzenöl oder -fett als Kohlenstoffquelle.

## Revendications

1. Micro-organisme, satisfaisant les exigences (1) à (3) suivantes :
(1) l'expression d'un gène phbA est réprimée ou une activité catalytique d'une enzyme codée par le gène est réprimée ;
(2) l'expression d'un gène bktB est amplifiée ou une activité catalytique d'une enzyme codée par le gène est augmentée ; et
(3) un gène de polyhydroxyalcanoate synthase et un gène de crotonyl-CoA réductase sont introduits à l'intérieur.

2. Micro-organisme selon la revendication 1,
dans lequel l'expression du gène phbA est réprimée ou l'activité catalytique de l'enzyme codée par le gène est réprimée, par au moins l'une des manipulations (1) à (6) suivantes :
(1) l'introduction d'un codon de terminaison supplémentaire entre le codon d'initiation et le codon de terminaison du gène phbA ;
(2) l'introduction d'une mutation qui provoque la réduction d'une activité de liaison aux ribosomes dans un site de liaison ribosomique du gène phbA ;
(3) l'introduction d'une mutation qui provoque la réduction de l'activité catalytique de l'enzyme codée par le gène phbA à l'intérieur du gène phbA ;
(4) l'utilisation d'une interférence d'ARN ;
(5) l'insertion d'un transposon à l'intérieur du gène phbA ; et
(6) la délétion d'une partie ou de la totalité du gène phbA.

3. Micro-organisme selon la revendication 1,
dans lequel l'expression du gène bktB est amplifiée par une insertion supplémentaire d'un promoteur doté d'une activité d'induction de la transcription du gène bktB en amont du gène bktB.

4. Micro-organisme selon la revendication 3,
dans lequel le promoteur supplémentaire inséré est l'ADN (1) ou (2) suivant :
(1) un ADN qui, en amont du gène bktB, présente une identité de séquence de 70
% ou plus avec un ADN de la séquence de bases de SEQ ID NO : 1 ou 2, et est doté d'une activité d'induction de la transcription du gène bktB ; et
(2) un ADN qui, en amont du gène bktB, s'hybride avec un ADN complémentaire de l'ADN de la séquence de bases de SEQ ID NO : 1 ou 2 dans des conditions stringentes, et est doté d'une activité d'induction de la transcription du gène bktB.

5. Micro-organisme selon la revendication 1,
dans lequel au moins l'un des gènes de polyhydroxyalcanoate synthase et/ou des gènes de crotonyl-CoA réductase (1) à (4) suivants est introduit :
(1) un gène qui présente une identité de séquence de 70 % ou plus avec un ADN de la séquence de bases de SEQ ID NO : 3, et code pour une enzyme dotée d'une activité de réduction du crotonyl-CoA pour produire du butyryl-CoA ;
(2) un gène qui s'hybride avec un ADN complémentaire de l'ADN de la séquence de bases de SEQ ID NO : 3 dans des conditions stringentes, et code pour une enzyme dotée d'une activité de réduction du crotonyl-CoA pour produire du butyryl-CoA ;
(3) un gène qui présente une identité de séquence de 70 % ou plus avec un ADN de la séquence de bases de SEQ ID NO : 4, et code pour une enzyme dotée d'une activité de synthèse d'un polyhydroxyalcanoate ; et
(4) un gène qui s'hybride avec un ADN complémentaire de l'ADN de la séquence de bases de SEQ ID NO : 4 dans des conditions stringentes, et code pour une enzyme dotée d'une activité de synthèse d'un polyhydroxyalcanoate.

6. Micro-organisme selon la revendication 5, qui est produit par recombinaison de gènes.

7. Micro-organisme selon l'une quelconque des revendications 1 à 5, qui est capable de produire un polyhydroxyalcanoate par utilisation d'une huile ou d'une graisse végétale en tant que source de carbone avec obtention d'une quantité de cellules sèches de 150 g/l ou plus, d'une teneur en polymère de 70 % ou plus, et d'une teneur en 3-hydroxyhexanoate de 12 % en mole ou plus.

8. Micro-organisme selon l'une quelconque des revendications 1 à 7,
dans lequel le polyhydroxyalcanoate est un polyester comportant du 3-hydroxybutyrate et du 3-hydroxyhexanoate en tant qu'unités structurales.

9. Micro-organisme selon l'une quelconque des revendications 1 à 8, qui est dérivé de Cupriavidus necator.

10. Micro-organisme selon la revendication 9, qui est dérivé de Cupriavidus necator H16.

11. Méthode de production d'un polyhydroxyalcanoate comportant une unité de 3-hydroxyhexanoate, la méthode comprenant :
la culture du micro-organisme selon l'une quelconque des revendications 1 à 10 avec une huile ou une graisse végétale en tant que source de carbone.
